(19) Europäisches Patentamt
European Patent Office
Office européen des brevets

(11) Veröffentlichungsnummer: **0 295 528**
**A2**

(12) **EUROPÄISCHE PATENTANMELDUNG**

(21) Anmeldenummer: **88109017.9**

(51) Int. Cl.⁴: **A61K 31/505 , A61K 31/54**

(22) Anmeldetag: **06.06.88**

(30) Priorität: **16.06.87 DE 3720074**

(43) Veröffentlichungstag der Anmeldung:
**21.12.88 Patentblatt 88/51**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Kurka, Peter, Dr.**
**Kölner Strasse 51**
**D-4010 Hilden(DE)**
Erfinder: **Goetze, Leopold, Dr.**
**Pahlkestrasse 17**
**D-5600 Wuppertal 1(DE)**

(54) **Mittel zur Verhinderung von Rangkämpfen bei Schweinen.**

(57) Die vorliegende Erfindung betrifft die Verwendung von 2-Pyrimidinyl-1-piperazin-Derivaten der Formel (I)

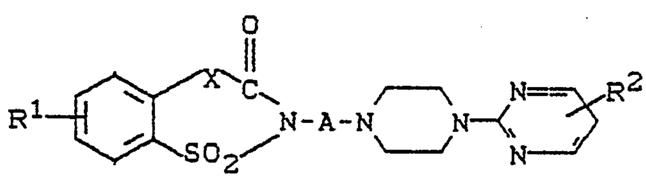

in welcher
$R^1$ für gleiche oder verschiedene Reste der Gruppen Wasserstoff, $C_{1-4}$-Alkyl, Halogen, Hydroxy, Nitro, $C_{2-4}$-Alkenyl, $C_{1-4}$-Alkoxy, $C_{1-4}$-Alkylthio, $C_{1-4}$-Halogenalkyl, $C_{1-4}$-Halogenalkoxy, $C_{1-4}$-Halogenalkylmercapto steht,
$R^2$ für gleiche oder verschiedene Reste der Gruppe Wasserstoff, Halogen, Hydroxy, Nitro, $C_{1-4}$-Alkyl, $C_{1-4}$-Alkoxy, $C_{1-4}$-Alkylmercapto, $C_{1-4}$-Halogenalkyl, $C_{1-4}$-Halogenalkoxy, $C_{1-4}$-Halogenalkylmercapto, $C_{2-4}$-Alkenyl steht,
X für eine direkte Bindung oder einen Rest $\geq$N-$R^3$ steht,
$R^3$ für Wasserstoff oder $C_{1-4}$-Alkyl steht,
A für einen geradkettigen oder verzweigten Alkylenrest mit bis zu 6 C-Atomen steht der gegebenenfalls substituiert sein kann,
sowie ihre physiologisch verträglichen Salze mit Säuren, zur Verhinderung von Rangkämpfen bei Schweinen.

EP 0 295 528 A2

## Mittel zur Verhinderung von Rangkämpfen bei Schweinen

Die vorliegende Erfindung betrifft Mittel zur Verhinderung von Rangkämpfen bei Schweinen sowie ihre Herstellung und Verwendung.

Zur Unterdrückung von Rangkämpfen beim Regrouping, d.h. beim Zusammenstellen neuer Gruppen (Transport, Umbuchtmaßnahmen) müssen beim Schwein Pharmaka eingesetzt werden, die eine dämpfende Wirkung auf die innerartliche Aggression besitzen um Teil- oder Totalverluste zu vermeiden. In dieser Indikation werden derzeit vorwiegend Neuroleptika (Phenothiazinderivate bzw. Butyrophenonderivate) verwendet, es wurde jedoch auch von Benzodiazepinderivaten (Diazepam) eine entsprechende Wirksamkeit nachgewiesen. Nachteile der ersteren bestehen in der Beeinflussung vegetativer Funktionen (Atemdepression, Hypothermie, Hypotension) sowie besonders beim Schwein im Auftreten paradoxer Reaktionen, während die Nachteile der Benzodiazepine in der mehr oder weniger starken Muskelrelaxation, der Amnesie und ebenfalls im Auftreten paradoxer Reaktionen zu sehen sind. Darüber hinaus kann die bei beiden Substanzklassen im Wirkungsprofil liegende Sedation der Schweine zu einer normalen oder verstärkten Kampftätigkeit beim oder nach Abklingen der effektiven Blutspiegel führen, da die Tiere erst dann in der Lage sind, sich kennenzulernen und eine Rangordnung herbeizuführen.

Auswahlkriterien für Stoffe, die in der obengenannten Indikation eingesetzt werden können, sind pharmakologische Testmodelle bei Labortieren (z.B.Kampfmaustest nach Tedeschi, Hemmung der territorialen Aggressivität der Ratte). Aus diesen Versuchen läßt sich jedoch kein zuverlässiger Schluß auf die Eignung von Verbindungen zur Unterdrückung von Rangkämpfen bei Schweinen ziehen. Zum einen können gerade bei Schweinen paradoxe Reaktionen auftreten, so daß der umgekehrte Effekt, nämlich eine Verstärkung von Rangkämpfen die Folge ist. Dieselbe nachteilige Wirkung kann bei zu starker sedativer Komponente im Wirkungsprofil der Substanz einen Einsatz unmöglich machen.

Weitere Nebeneffekte bzw. unerwünschte Wirkungen (Beeinträchtigung vegetativer Funktionen, deutliche Myorelaxation) können sich besonders beim Transport der Tiere negativ auswirken.

Es wurde nun gefunden, daß sich die 2-Pyrimidinyl-1-piperazin-Derivate der Formel (I)

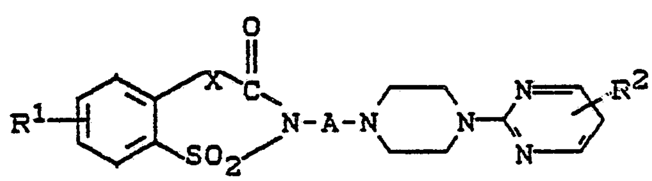

in welcher

$R^1$ für gleiche oder verschiedene Reste der Gruppen Wasserstoff, $C_{1-4}$-Alkyl, Halogen, Hydroxy, Nitro, $C_{2-4}$-Alkenyl, $C_{1-4}$-Alkoxy, $C_{1-4}$-Alkylthio, $C_{1-4}$-Halogenalkyl, $C_{1-4}$-Halogenalkoxy, $C_{1-4}$-Halogenalkylmercapto steht,

$R^2$ für gleiche oder verschiedene Reste der Gruppe Wasserstoff, Halogen, Hydroxy, Nitro, $C_{1-4}$-Alkyl, $C_{1-4}$-Alkoxy, $C_{1-4}$-Alkylmercapto, $C_{1-4}$-Halogenalkyl, $C_{1-4}$-Halogenalkoxy, $C_{1-4}$-Halogenalkylmercapto, $C_{2-4}$-Alkenyl steht,

X für eine direkte Bindung oder einen Rest $\geq$N-$R^3$ steht,

$R^3$ für Wasserstoff oder $C_{1-4}$-Alkyl steht,

A für einen geradkettigen oder verzweigten Alkylenrest mit bis zu 6C-Atomen steht der gegebenenfalls substituiert sein kann,

sowie ihre physiologisch verträglichen Salze mit Säuren, hervorragend zur Unterdrückung von Rangkämpfen beim Zusammenstellen von Gruppen von Schweinen eignen.

Die Verbindungen der Formel (I) und ihre Herstellung sind bekannt aus EP-OS 129 128.

Bevorzugt sind Verbindungen der Formel (I), in der

$R^1$ für Wasserstoff, Halogen, insbesondere Chlor oder Fluor, Methyl, Ethyl, Methoxy, Ethoxy, Methylendioxy, Ethylendioxy steht;

$R^2$ für Wasserstoff, Halogen, insbesondere Chlor oder Fluor steht,

X für eine direkte Bindung oder den Rest $\geq$N-$R^3$ steht,

$R^3$ für Wasserstoff oder $C_{1-4}$-Alkyl, insbesondere Methyl oder Ethyl steht,

A für einen geradkettigen Alkylenrest mit 3 bis 6 C-Atomen insbesondere n-Propylen oder n-Butylen steht.

Besonders bevorzugt sind Verbindungen der Formel (I), in welcher
$R^1$ für Wasserstoff, Fluor, Chlor oder Methoxy steht,
$R^2$ für Wasserstoff oder Fluor steht,
X für eine direkte Bindung oder einen Rest $>N-CH_3$ steht,
A für $-(CH_2)_3-$ oder $-(CH_2)_4-$ steht.

Im einzelnen seien folgende Verbindungen genannt:

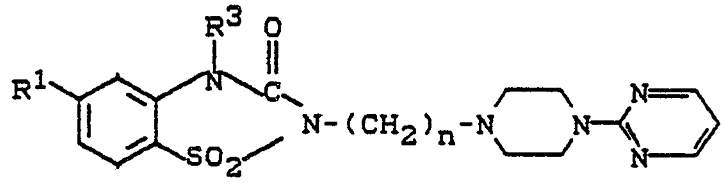

| $R^1$ | $R^3$ | n |
|---|---|---|
| H | H | 3 |
| H | $CH_3$ | 3 |
| H | H | 4 |
| H | $CH_3$ | 4 |
| Cl | H | 3 |
| Cl | $CH_3$ | 3 |
| $OCH_3$ | H | 3 |
| $OCH_3$ | $CH_3$ | 3 |

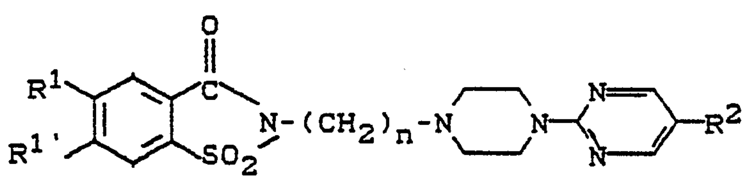

| $R^1$ | $R^{1'}$ | $R^2$ | n |
|---|---|---|---|
| Cl | H | H | 4 |
| H | Cl | H | 4 |
| F | H | H | 4 |
| F | H | H | 3 |
| H | H | H | 3 |
| H | H | H | 4 |
| H | H | F | 4 |

Besonders hervorgehoben sei 2-(4-(4-(2-Pyrimidinyl)-1-piperazinyl-propyl-1,2-benzisothiazol-3(2H)on-1,1-dioxid und 2-(4-(4-Pyrimidinyl)-1-piperazinyl-1,2-benzisothiazol-3(2H)on-1,1-dioxid (Ipsapirone).

Als Säuren mit denen die Verbindungen der Formel (I) Salze bilden können seien anorganische und organische Säuren genannt. Zu den anorganischen Säuren gehören Salzsäure, Schwefelsäure, Phosphorsäure.Zu den organischen Säuren gehören Ameisensäure, Essigsäure.

Die Wirkstoffe werden in Form von für Tiere geeigneten Zubereitungen angewandt.

Für Tiere geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele; Emulsionen und Suspensionen zur oralen oder kutanen Anwendung sowie zur Injektion; feste Zubereitungen wie Pulver, Premixe oder Konzentrate, Granulate, Pellets, Tabletten, Boli, Kapseln; Aerosole und Inhalate.

Injektionslösungen werden intravenös, intramuskulär und subcutan verabreicht. Es werden 0,1 bis 50 mg Wirkstoff, bevorzugt 0,2 bis 10 mg Wirkstoff pro kg Körpergewicht der Tiere eingesetzt. Die Lösungen enthalten 0,1 bis 10 %, bevorzugt 0,2 bis 5 % Wirkstoff.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, Ester wie Milchsäureethylester, N-Methyl-pyrrolidon, sowie Gemische derselben

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut oder in Körperhöhlen werden aufgeträufelt, aufgestrichen, eingerieben, aufgesprüht, gebadet. Diese Lösungen werden wie oben bei den Injektionslösungen beschrieben hergestellt. Bei der Herstellung können Verdickungsmittel zugefügt werden.

Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß Formulierungen werden hergestellt, indem der Wirkstoff in geeigneten hautveträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Milchsäureethylester, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind Dimethylsulfoxid (DMSO), spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate,

Gelatine.

Emulsionen können oral, kutan oder als Injektionen angewendet werden.

Emulsionen sind entweder vom Typ Wasser in Öl oder vom Typ Öl in Wasser.

Sie werden hergestellt, indem man den Wirkstoff in einer Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono-und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt: Wasser,Alkohole wie z.B. Propylenglycol, Glycerin, Sorbitol und ihre Gemische.

Als Emulgatoren seien genannt: Tenside (beinhaltet Emulgatoren und Netzmittel), wie

1. nichtionogene, z.B. polyoxyethyliertes Rizinusöl, polyoxyethyliertes Sorbitan-monoleat, Sorbitan Monostearat, Glycerinmonostearat, Polyoxyethylstearat, Alkylphenolpolyglykolether,

2. ampholytische wie Di-Na-N-lauryl-ß-iminodipropionat oder Lecithin,

3. anionaktive, wie Na-Laurylsulfat, Fettalkoholethersulfate, Mono/Dialkylpolyglykoletherorthophosphorsäureester-Monoethanolaminsalz,

4. kationaktive wie Cetyltrimethylammoniumchlorid.

Als weitere Hilfsstoffe sind geeignet: viskositätserhöhende und die Emulsion stabilisierende Stoffe wie Carboxymethylcellulose,Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, kutan oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien genannt alle homogenen Lösungsmittel und Lösungsmittelgemische.

Als Netzmittel (Dispergiermittel) seien genannt:

Tenside (beinhaltet Emulgatoren und Netzmittel) wie

1. anionaktive, wie Na-Laurylsulfat, Fettalkoholethersulfate, Mono/Dialkylpolyglykoletherorthophosphorsäureester-Monoethanolaminsalz, Ligninsulfonate oder Dioctylsulfosuccinat,

2. kationaktive wie Cetyltrimethylammoniumchlorid,

3. ampholytische wie Di-Na-N-lauryl-ß-iminodipropionat oder Lecithin

4. nicht ionogene, z.B. polyoxethyliertes Rizinusöl, polyoxyethyliertes Sorbitan-Monoleat, Sorbitan Monostearat, Ethylalkohol, Glycerinmonostearat, Polyoxyethylenstearat,Alkylphenolpolyglykolether, Pluronic®.

Als weitere Hilfsstoffe seien die weiter oben angegebenen genannt.

Halbfeste Zubereitungen können oral oder kutan verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt

worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in Form ihrer oben erwähnten festen oder flüssigen Formulierungen auch eingekapselt vorliegen.

Die Wirkstoffe können auch in Form eines Aerosols angewendet werden. Dazu wird der Wirkstoff in geeigneter Formulierung unter Druck fein verteilt.

Es kann auch vorteilhaft sein, die Wirkstoffe in Formulierungen anzuwenden, die den Wirkstoff verzögert freigeben.

Die Verabreichung der Wirkstoffe erfolgt bevorzugt zusammen mit dem Futter und/oder dem Trinkwasser.

Zum Futter zählen Einzelfuttermittel pflanzlicher Herkunft wie Heu, Rüben, Getreide, Getreidenebenprodukte, Einzelfuttermittel tierischer Herkunft wie Fleisch, Fette, Milchprodukte, Knochenmehl, Fischprodukte, ferner die Einzelfuttermittel wie Vitamine, Proteine, Aminosäuren, z.B. DL-Methionin, Salze wie Kalk und Kochsalz. Zum Futter zählen auch Ergänzungs-, Fertig- und Mischfuttermittel. Diese enthalten Einzelfuttermittel in einer Zusammensetzung die eine ausgewogene Ernährung hinsichtlich der Energie-und Proteinversorgung sowie der Versorgung mit Vitaminen, Mineralsalzen und Spurenelementen sicherstellen.

Die Konzentration der Wirkstoffe im Futter beträgt normalerweise etwa 0,01 - 500 ppm, bevorzugt 0,1 - 50 ppm.

Die Wirkstoffe können als solche oder in Form von Prämixen oder Futterkonzentraten dem Futter zugesetzt werden.

Prämixe und Futterkonzentrate sind Mischungen des Wirkstoffes mit einem geeigneten Trägerstoff.

Zu den Trägerstoffen zählen die Einzelfuttermittel oder Gemische derselben.

Sie können darüber hinaus weitere Hilfsmittel enthalten, wie z.B.- Substanzen, die die Fließfähigkeit und Mischbarkeit regulieren, wie z.B. Kieselsäuren, Bentonite, Ligninsulfonate. Darüber hinaus können Antioxydantien wie BHT oder Konservierungsmittel wie Sorbinsäure oder Calciumpropionat hinzugefügt sein.

Konzentrate zur Applikation über das Trinkwasser müssen so formuliert sein, daß beim Vermischen mit dem Trinkwasser eine klare Lösung oder eine stabile homogene Suspension entsteht.

Als Trägerstoffe sind daher wasserlösliche Substanzen (Futterzusatzmittel) wie Zucker oder Salze (z.B. Citrate, Phosphate, Kochsalz, Na-carbonat) geeignet.

Sie können ebenfalls Antioxydantien und Konservierungsmittel enthalten.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

## Beispiel A

15 Ferkel im Alter von 4 Wochen aus 2 Würfen erhielten jeweils 2 ml einer 0,25 %igen wäßrigen Lösung $\triangleq$ 0,6 mg Wirkstoff pro kg Körpergewicht, von 2-(4-(4-(2-Pyrimidinyl)-1-piperazinyl)-propyl)-1,2-benzisothiazol-3(2H)on-1,1-dioxid-hydrochlorid intramuskulär injiziert. Die zuvor nach Würfen getrennten Tiere wurden zusammengebracht und über 2 Stunden beobachtet. Die Tiere fraßen zusammen, Rangkämpfe traten nicht auf.

## Beispiel B

Der Versuch des Beispiels A wurde wiederholt mit 19 Ferkeln im Alter von 7 Wochen aus 2 Würfen. Den Tieren wurden 2 ml einer 2 %igen wäßrigen Lösung von 2-(4-(4-(2-Pyrimidinyl)-1-piperazinyl)propyl)-1,2-benzisothiazol-3(2H)on-1,1-dioxid-hydrochlorid intramuskulär injiziert. Die zuvor nach Würfen getrennten Tiere wurden zusammengebracht und über 2 Stunden beobachtet. Rangkämpfe traten nicht auf.

Beispiel C

Der Versuch des Beispiels A wurde mit 10 Ferkeln im Alter von 7 Wochen, die aus 4 Würfen zusammengestellt waren, wiederholt. Die Tiere erhielten 2 ml einer 0,5 %igen wäßrigen Lösung von Ipsapirone-Hydrochlorid (≙ 0,7 mg pro kg Körpergewicht) intramuskulär injiziert bevor sie zusammengebracht wurden. Rangkämpfe wurden nicht beobachtet.

Beispiel 1

Darstellung von 2-(4-(4-(2-Pyrimidinyl)-1-piperazinyl)butyl)-1,2-benzoisothiazol-3(2H)on-1,1-dioxid

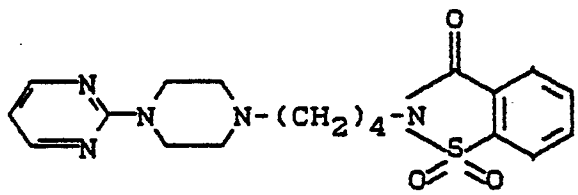

0,02 Mol 2-(4-Brombutyl)-1,2-benzoisothiazol-3-(2H)on-1,1-dioxid und 0,02 Mol 1-(2-Pyrimidinyl)-piperazin werden mit 0,02 Mol $K_2CO_3$ in 150 ml absolutem Dimethylformamid (DMF) 1Stunde bei 100 °C gerührt. Danach engt man ein. Mit Wasser versetzt, wird die organische Substanz in Methylenchlorid ($CH_2Cl_2$) aufgenommen. Die getrocknete $CH_2Cl_2$-Phase wird auf eine Kieselgelsäule gegeben und mit $CH_2Cl_2/CH_3OH$ (95:5) eluiert.
Ausbeute: 34 % der Theorie; Fp.: 138-139 °C.

**Ansprüche**

1. Verwendung von 2-Pyrimidinyl-1-piperazin-Derivaten der Formel (I)

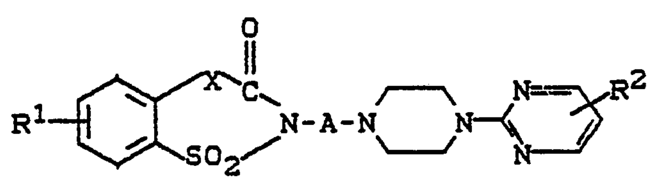

in welcher
$R^1$ für gleiche oder verschiedene Reste der Gruppen Wasserstoff, $C_{1-4}$-Alkyl, Halogen, Hydroxy, Nitro, $C_{2-4}$-Alkenyl, $C_{1-4}$-Alkoxy, $C_{1-4}$-Alkylthio, $C_{1-4}$-Halogenalkyl, $C_{1-4}$-Halogenalkoxy, $C_{1-4}$-Halogenalkylmercapto steht,
$R^2$ für gleiche oder verschiedene Reste der Gruppe Wasserstoff, Halogen, Hydroxy, Nitro, $C_{1-4}$-Alkyl, $C_{1-4}$-Alkoxy, $C_{1-4}$-Alkylmercapto, $C_{1-4}$-Halogenalkyl, $C_{1-4}$-Halogenalkoxy, $C_{1-4}$-Halogenalkylmercapto, $C_{2-4}$-Alkenyl steht,
X für eine direkte Bindung oder einen Rest $\geq$N-$R^3$ steht,
$R^3$ für Wasserstoff oder $C_{1-4}$-Alkyl steht,
A für einen geradkettigen oder verzweigten Alkylenrest mit bis zu 6 C-Atomen steht der gegebenenfalls substituiert sein kann,
sowie ihrer physiologisch verträglichen Salze mit Säuren, zur Unterdrückung von Rangkämpfen beim Zusammenstellen von Gruppen von Schweinen.

2. Mittel zur Unterdrückung von Rangkämpfen beim Zusammenstellen von Gruppen von Schweinen, gekennzeichnet durch einen Gehalt an 2-Pyrimidinyl-1-piperazin-Derivate der Formel (I)

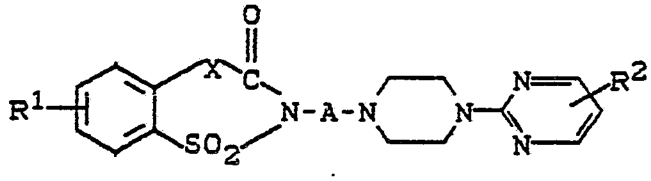

in welcher
$R^1$ für gleiche oder verschiedene Reste der Gruppen Wasserstoff, $C_{1-4}$-Alkyl, Halogen, Hydroxy, Nitro, $C_{2-4}$-Alkenyl, $C_{1-4}$-Alkoxy, $C_{1-4}$-Alkylthio, $C_{1-4}$-Halogenalkyl, $C_{1-4}$-Halogenalkoxy, $C_{1-4}$-Halogenalkylmercapto steht,
$R^2$ für gleiche oder verschiedene Reste der Gruppe Wasserstoff, Halogen, Hydroxy, Nitro, $C_{1-4}$-Alkyl, $C_{1-4}$-Alkoxy, $C_{1-4}$-Alkylmercapto, $C_{1-4}$-Halogenalkyl, $C_{1-4}$-Halogenalkoxy, $C_{1-4}$-Halogenalkylmercapto, $C_{2-4}$-Alkenyl steht,
X für eine direkte Bindung oder einen Rest $>$N-$R^3$ steht,
$R^3$ für Wasserstoff oder $C_{1-4}$-Alkyl steht,
A für einen geradkettigen oder verzweigten Alkylenrest mit bis zu 6 C-Atomen steht der gegebenenfalls substituiert sein kann,
sowie ihrer physiologisch verträglichen Salze mit Säuren.

3. Verwendung von 2-Pyrimidinyl-1-piperazin-Derivaten der Formel (I)

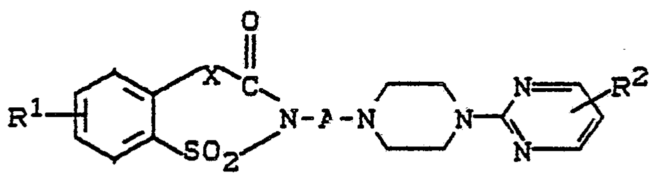

in welcher
$R^1$ für gleiche oder verschiedene Reste der Gruppen Wasserstoff, $C_{1-4}$-Alkyl, Halogen, Hydroxy, Nitro, $C_{2-4}$-Alkenyl, $C_{1-4}$-Alkoxy, $C_{1-4}$-Alkylthio, $C_{1-4}$-Halogenalkyl, $C_{1-4}$-Halogenalkoxy, $C_{1-4}$-Halogenalkylmercapto steht,
$R^2$ für gleiche oder verschiedene Reste der Gruppe Wasserstoff, Halogen, Hydroxy, Nitro, $C_{1-4}$-Alkyl, $C_{1-4}$-Alkoxy, $C_{1-4}$-Alkylmercapto, $C_{1-4}$-Halogenalkyl, $C_{1-4}$-Halogenalkoxy, $C_{1-4}$-Halogenalkylmercapto, $C_{2-4}$-Alkenyl steht,
X für eine direkte Bindung oder einen Rest $>$N-$R^3$ steht,
$R^3$ für Wasserstoff oder $C_{1-4}$-Alkyl steht,
A für einen geradkettigen oder verzweigten Alkylenrest mit bis zu 6 C-Atomen steht der gegebenenfalls substituiert sein kann,
sowie ihrer physiologisch verträglichen Salze mit Säuren zur Herstellung von Mitteln zur Unterdrückung von Rangkämpfen beim Zusammenstellen von Gruppen von Schweinen.